# EUROPÄISCHE PATENTSCHRIFT

(11) **EP 0 790 226 B1**
(45) Veröffentlichungstag und Bekanntmachung des Hinweises auf die Patenterteilung: **26.04.2000**
(21) Anmeldenummer: 96119915.5
(22) Anmeldetag: 12.12.1996
(51) Int. Cl.: C07C 29/151, C07C 31/04

(54) **Verfahren zum Erzeugen von Methanol**
Process for the production of methanol
Procédé pour la production de méthanol

(30) Priorität: 15.02.1996 DE 19605572
(43) Veröffentlichungstag der Anmeldung: 20.08.1997
(73) Patentinhaber: Metallgesellschaft Aktiengesellschaft, 60325 Frankfurt am Main (DE)
(72) Erfinder: König, Peter, Dr., 60439 Frankfurt am Main (DE); Göhna, Hermann, 65812 Bad Soden (DE)

(56) Entgegenhaltungen:
- EP-A- 0 047 345
- DE-A- 4 416 425

## Beschreibung

Die Erfindung betrifft ein Verfahren zum Erzeugen von Methanol aus einem Wasserstoff und Kohlenoxide enthaltenden Synthesegas durch Umsetzung an körnigen, kupferhaltigen Katalysatoren bei Drücken im Bereich von 20 bis 120 bar und Temperaturen im Bereich von 130 bis 350°C, wobei man das Synthesegas durch mindestens zwei in Serie geschaltete Synthese-Reaktoren leitet.

Ein Verfahren dieser Art ist in DE-A-4416425 beschrieben. Hierbei leitet man das Synthesegas zunächst durch einen adiabatisch betriebenen Schachtreaktor und dann durch einen wassergekühlten Röhrenreaktor. Beide Reaktortypen, der Schachtreaktor und der Röhrenreaktor, gehören zur bekannten Technik der katalytischen Erzeugung von Methanol.

Der Erfindung liegt die Aufgabe zugrunde, auf der Basis des Röhrenreaktors ein kostengünstiges Verfahren bereitzustellen. Hierbei soll es möglich sein, den wassergekühlten Röhrenreaktor möglichst klein auszubilden. Beim eingangs genannten Verfahren wird die Aufgabe erfindungsgemäß dadurch gelöst,
a) daß man frisches und zurückgeführtes Synthesegas, vorgewärmt auf eine Temperatur im Bereich von 220 bis 280°C, in den ersten Synthese-Reaktor leitet, in welchem der Katalysator in Röhren angeordnet ist, die von unter erhöhtem Druck siedendem Wasser als Kühlmittel umgeben sind,
b) daß man im ersten Synthese-Reaktor 40 bis 80% des eingeleiteten Kohlenoxide umsetzt und ein Gase und Methanoldampf enthaltendes erstes Gemisch abzieht,
c) daß man das erste Gemisch durch mindestens einen zweiten Synthese-Reaktor leitet, in welchem der Katalysator mit Synthesegas gekühlt wird, daß man ein Gase und Methanoldampf enthaltendes zweites Gemisch aus dem zweiten Synthese-Reaktor abzieht, das zweite Gemisch kühlt und Methanoldampf kondensiert, und
d) daß man vom methanolhaltigen Kondensat zurückzuführendes Synthesegas abtrennt, das abgetrennte Synthesegas mindestens teilweise als Kühlmittel durch den zweiten Synthese-Reaktor leitet und das dabei vorgewärmte Synthesegas in den ersten Reaktor zurückführt, wobei man dem zurückzuführenden Synthesegas aus einer externen Quelle kommendes frisches Synthesegas zumischt.

Im zweiten Synthese-Reaktor wird der Katalysator im allgemeinen durch das als Kühlmittel dienende Synthesegas indirekt gekühlt. Es ist aber auch eine direkte Kühlung möglich, wobei man einen Teilstrom des zurückzuführenden Synthesegases abzweigt und es dem aus dem ersten Reaktor kommenden ersten Gemisch zugibt, wobei das Gemisch gekühlt wird.

Beim erfindungsgemäßen Verfahren kann der erste Synthese-Reaktor erheblich kleiner als bei bekannten Verfahren mit nur einem einzigen Reaktor gebaut werden. Im wassergekühlten ersten Reaktor kann man beim erfindungsgemäßen Verfahren den Umsatz bei relativ hohen Temperaturen bewirken, wodurch vorteilhafterweise ein höhergespannter Wasserdampf erzeugt wird. Gleichzeitig tritt das erste Gemisch aus dem ersten Reaktor mit relativ hoher Temperatur aus und wird ohne Zwischenkühlung direkt in den zweiten Synthesereaktor geleitet. In dem im Gegenstrom mit Synthesegas gekühlten zweiten Synthese-Reaktor liegen die Temperaturen zum Ausgang hin ziemlich niedrig, was für die Methanolsynthese vorteilhaft ist. Im zweiten Reaktor kann der Katalysator in Röhren oder Kammern angeordnet sein, die vom gasförmigen Kühlmittel umströmt sind. Ferner ist im zweiten Reaktor eine Katalysator-Schüttung möglich, die von Kühlröhren durchzogen ist, durch welche das Synthesegas strömt. Der zweite Reaktor kann auch aus mehreren, in Serie geschalteten Teil-Reaktoren gebildet sein.

Vorteilhafterweise besteht das in den ersten Sythese-Reaktor geleitete Synthesegas zu 15 bis 40 Vol.-% aus frischem Synthesegas. Dieser relativ hohe Anteil an frischem Synthesegas bedeutet auch, daß nur eine relativ geringe Menge an Synthesegas zurückgeführt wird, wodurch sich die dafür nötige Kompression verbilligt.

Die zu verwendenden Katalysatoren sind bekannt und handelsüblich. Neben CuO enthalten sie z.B. noch ZnO und Al₂O₃.

Die im ersten und zweiten Reaktor enthaltenen Katalysatormengen werden üblicherweise ein Gewichtsverhältnis von 3 : 2 bis 1 : 4 aufweisen. Vorzugsweise liegt dieses Gewichtsverhältnis im Bereich von 1 : 1 bis 1 : 3. Es ist also durchaus möglich, daß der zweite Reaktor eine größere Katalysatormenge als der erste Reaktor enthält. Es hat sich gezeigt, daß die gesamte Katalysatormenge etwa gleich oder nur geringfügig höher ist als bei bekannten Methanolsynthese-Verfahren, die allein nur mit dem mit Wasser gekühlten Röhrenreaktor arbeiten.

Da der erste Synthese-Reaktor nicht für einen möglichst hohen Umsatz an Kohlenoxiden sorgen muß, kann man diesen ersten Reaktor mit einer großen Menge an Synthesegas beaufschlagen. Üblicherweise liegt die Gasbelastung für diesen ersten Reaktor pro Stunde und pro m³ Katalysator im Bereich von 14000 bis 24000 Nm³. Das aus dem ersten Synthese-Reaktor abgezogene erste Gemisch enthält üblicherweise 4 bis 10 Vol.-% Methanoldampf. Das aus dem zweiten Reaktor abgezogene zweite Gemisch hat zumeist Temperaturen im Bereich von 130 bis 240°C.

Ausgestaltungsmöglichkeiten des Verfahrens werden mit Hilfe der Zeichnung erläutert. Es zeigt:
- Fig. 1: ein Fließschema des Verfahrens,
- Fig. 2: eine kompakte Bauweise der beiden Synthese-Reaktoren und
- Fig. 3: eine weitere Variante der beiden Reaktoren.

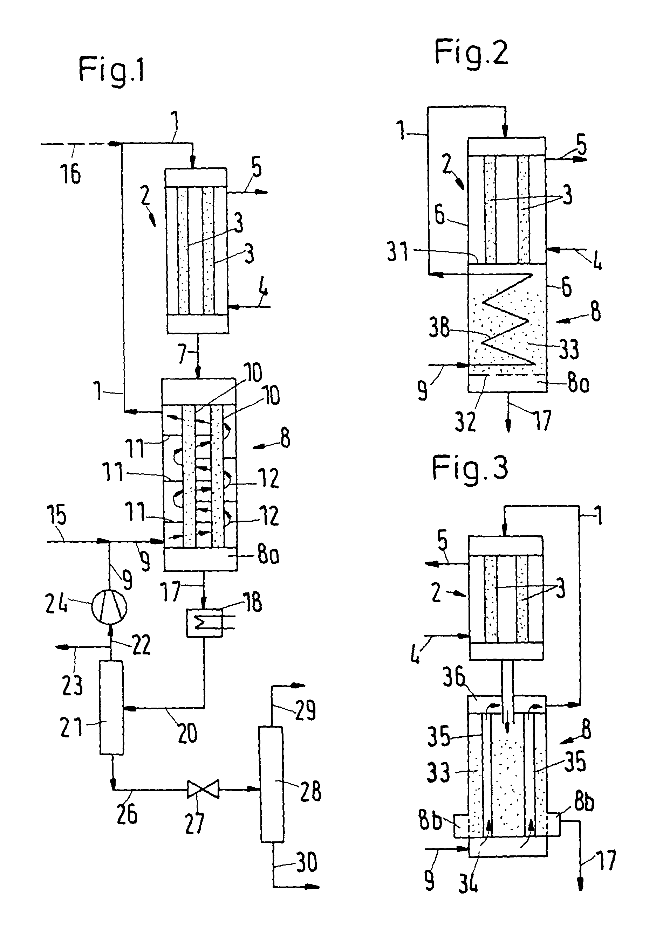

Ein Gemisch aus frischem und zurückgeführtem Synthesegas wird gemäß Fig. 1 durch die Leitung (1) in den ersten Synthese-Reaktor (2) geführt. Dieser erste Reaktor ist ein an sich bekannter Röhrenreaktor, in welchem der Kupferkatalysator in Röhren (3) angeordnet ist. Als Kühlmittel dient unter erhöhtem Druck siedendes Wasser, das in der Leitung (4) herangeführt wird. Ein Gemisch aus siedendem Wasser und Wasserdampf zieht man in der Leitung (5) ab und führt es zu einer nicht dargestellten, an sich bekannten Dampftrommel. Das in den Reaktor (2) eintretende Synthesegas ist auf eine Temperatur im Bereich von 220 bis 280°C vorgewärmt, der Druck liegt im Bereich von 2 bis 12 MPa (20 bis 120 bar) und zumeist im Bereich von 4 bis 10 MPa (40 bis 100 bar). Das Kühlmittel, das man in der Leitung (5) abzieht, hat üblicherweise eine Temperaur im Bereich von 240 bis 280°C.

Im ersten Reaktor (2) werden 40 bis 80% der durch die Leitung (1) in den Reaktor gegebenen Kohlenoxide umgesetzt. Aus dem Reaktor (2) zieht man in der Leitung (7) ein erstes Gemisch ab, welches aus Gasen und Dämpfen besteht, wobei der Methanol-Gehalt 4 bis 10 Vol.-% und zumeist 5 bis 8 Vol.-% beträgt. Ohne das erste Gemisch zu kühlen, leitet man es direkt in den zweiten Synthese-Reaktor (8), der hier ebenfalls als Röhrenreaktor ausgestaltet ist. Als Kühlmedium dient im Reaktor (8) Synthesegas, das in der Leitung (9) herangeführt wird. Um die Kühlung der den Kupferkatalysator enthaltenden Röhren (10) zu intensivieren, weist der vom Kühlgas durchströmte Raum im Reaktor (8) Leitbleche (11) auf. Diese Bleche (11) bewirken einen gewundenen Strömungsweg des Kühlgases, der durch die Pfeile (12) angedeutet ist. Das als Kühlmittel dienende Synthesegas wird im Reaktor (8) vorgewärmt und strömt dann durch die Leitung (1) zum ersten Synthese-Reaktor (2). Frisches Synthesegas, das man in einer an sich bekannten, nicht dargestellten Anlage erzeugt, wird in der Leitung (15) herangeführt und dem zurückzuführenden Synthesegas zugemischt. Möglich ist auch, frisches Synthesegas durch die Leitung (16) heranzuführen und es der Leitung (1) zuzugeben. Es wird dafür gesorgt, daß das Synthesegas, welches in den ersten Reaktor (2) eintritt, Wasserstoff und Kohlenoxide etwa in folgenden Anteilen aufweist:
H₂ = 40 bis 80 Vol.-%,
CO = 3 bis 15 Vol.-% und
CO₂ = 1 bis 10 Vol.-%.

Im gasgekühlten Reaktor (8) liegen die Temperaturen im Katalysator in der Nähe der Austrittskammer (8a) im Bereich von 130 bis 240°C und üblicherweise im Bereich von 160 bis 220°C. Durch diese relativ niedrigen Temperaturen wird die Bildung von Methanol in dem durch den Katalysator strömenden Gasgemisch begünstigt.

Ein Gase und Methanoldampf enthaltenden Produktgemisch, das hier auch als zweites Gemisch bezeichnet wird, verläßt den Reaktor (8) durch die Leitung (17) und strömt durch den indirekten Kühler (18), wobei Methanol kondensiert wird. Anschließend gibt man das Gemisch durch die Leitung (20) in einen ersten Abscheidebehälter (21), in welchem sich Gase und Flüssigkeit trennen. Die Gase werden durch die Leitung (22) abgezogen, wobei man einen Teil durch die Leitung (23) aus dem Verfahren entfernt. Mit Hilfe des Verdichters (24) führt man die Gase als zurückzuführendes Synthesegas in der bereits beschriebenen Weise zunächst durch die Leitung (9) in den Reaktor (8).

Methanol enthaltende Flüssigkeit zieht man aus dem ersten Abscheidebehälter (21) durch die Leitung (26) ab und führt die Flüssigkeit durch ein Entspannungsventil (27) zu einem zweiten Abscheidebehälter (28). Man zieht daraus durch die Leitung (29) ein Restgas ab und erhält in der Leitung (30) Rohmethanol, das nun noch in nicht dargestellter, an sich bekannter Weise destillativ gereinigt wird.

Fig. 2 zeigt eine kompakte Bauweise für die beiden Reaktoren (2) und (8), die in einem gemeinsamen Gehäuse (6) untergebracht sind. Dazwischen befindet sich der flüssigkeitsdichte Trennboden (31). Der zweite Reaktor (8) weist auf einem Rost (32) eine Katalysator-Schüttung (33) auf, durch die sich eine Kühlmittelleitung (38) zieht. Das von den Röhren (3) kommende erste Gemisch strömt durch diese Schüttung abwärts zur Kammer (8a). Die übrigen Bezugsziffern haben die bereits beschriebene Bedeutung, das gilt auch für Fig. 3. Hier enthält der zweite Reaktor (8) die Katalysator-Schüttung (33), durch die das vom ersten Reaktor (2) kommende, Gase und Methanoldampf enthaltende erste Gemisch strömt. Das zweite Gemisch gelang zunächst in die außenliegende Sammelkammer (8b) und wird in der Leitung (17) abgezogen. Das in der Leitung (9) herangeführte Synthesegas tritt zunächst in eine Verteilkammer (34) ein und strömt dann aufwärts als Kühlgas durch die Röhren (35). Gesammelt in der Kammer (36) gelangt das Synthesegas durch die Leitung (1) in den ersten Reaktor (2).

### Beispiele:

Der erfindungsgemäßen Arbeitsweise der Beispiele 2 und 4 liegt die Anordnung der Fig. 1 zugrunde, aber ohne die Leitung (16). In den Vergleichsbeispielen 1 und 3 wird nur mit dem siedewassergekühlten Reaktor (2) und ohne den mit Synthesegas gekühlten Reaktor (8) gearbeitet. Die Daten der Beispiele sind teilweise berechnet. Der in allen Fällen verwendete Katalysator ist handelsüblich und besteht aus 60 Gew.-% CuO, 30 Gew.-% ZnO und 10 Gew.-% Al₂O₃. In der nachfolgenden Tabelle I bedeutet "Leitung (15)" die Zusammensetzung des frischen Synthesegases, das in der Leitung (15) herangeführt wird, und "Leitung (1)" die Zusammensetzung des in der Leitung (1) in den Reaktor (2) eintretenden Synthesegases. Die Menge an frischem Synthesegas ist in allen Beispielen gleich, die Daten sind auf 1 Kmol/h bezogen.

| Tabelle I: | Beispiel 1 | Beispiel 2 | Beispiel 3 | Beispiel 4 |
|---|---|---|---|---|
| Leitung (15): | | | | |
| H₂ (Mol-%) | 70,7 | 70,7 | 68,1 | 68,1 |
| CO (Mol-%) | 17,2 | 17,2 | 21,0 | 21,0 |
| CO₂ (Mol-%) | 7,9 | 7,9 | 7,7 | 7,7 |
| Inerte (Mol-%) | 4,2 | 4,2 | 3,2 | 3,2 |

| Leitung (1): | | | | |
|---|---|---|---|---|
| H₂ (Mol-%): | 74,6 | 74,5 | 61,3 | 61,7 |
| CO (Mol-%) | 5,3 | 5,7 | 5,9 | 6,5 |
| CO₂ (Mol-%) | 4,3 | 4,5 | 4,8 | 5,1 |
| CH₃OH (Mol-%) | 0,3 | 0,3 | 0,5 | 0,5 |
| Sonstige (Mol-%) | 15,5 | 15,0 | 27,5 | 26,2 |
| Temperatur | 220°C | 225°C | 230°C | 235°C |
| Druck | 70 bar | 70 bar | 81 bar | 81 bar |

Die gesamte Menge am Katalysator ist im Beispiel 2, das mit zwei Synthesereaktoren (2) und (8) arbeitet, nur um das 1,06-fache höher als im Vergleichsbeispiel 1 mit seinem einzigen Reaktor (2). Im Beispiel 2 wird die gesamt Katalysatormenge auf die Reaktoren (2) und (8) im Verhältnis 40 : 60 verteilt. Im Beispiel 1 beträgt das Mengenverhältnis ("Kreislaufverhältnis") von frischem Synthesegas in der Leitung (15) zu dem vom Kompressor (24) kommenden, zurückzuführenden Synthesegas 1:3,3, im Beispiel 2 liegt das Kreislaufverhältnis bei 1:2,5, so daß eine geringere Gasmenge durch die Leitung (1) geführt wird.

Im Beispiel 1 wird beim Kühlen das Röhrenreaktors Sattdampf von 4 MPa (40 bar) erzeugt, wogegen man gemäß Beispiel 2 durch die Leitung (5) Sattdampf von 5 MPa (50 bar) abführen kann. Der Katalysator wird im Beispiel 1 pro m³ und pro Stunde mit 11 000 Nm³ Synthesegas belastet, demgegenüber erreicht man im Röhrenreaktor (2) des Beispiels 2 eine Belastung von 20 000 Nm³/m³/h. Im Beispiel 3 (Vergleichsbeispiel) beträgt die Belastung 12 000 Nm³/m³/h und im erfindungsgemäßen Beispiel 4 arbeitet man im Röhrenreaktor (2) mit einer Belastung von 18 000 Nm³/m³/h. Das Kreislaufverhältnis beträgt im Beispiel 3 1:4 und im Beispiel 4 1:2,7.

Die Tabelle II zeigt die Konzentration einiger wichtiger Komponenten des Gas- und Dampfgemisches am Austritt des Reaktors (2) und am Austritt des Reaktors (8):

| Tabelle II | Beispiel 1 | Beispiel 2 | Beispiel 3 | Beispiel 4 |
|---|---|---|---|---|
| Austritt Reaktor (2): | | | | |
| CH₃OH (Mol-%) | 6,6 | 5,0 | 6,8 | 5,7 |
| CO₂ (Mol-%) | 3,0 | 3,5 | 3,8 | 4,4 |
| CO (Mol-%) | 1,6 | 3,0 | 2,0 | 3,3 |
| H₂ (Mol-%) | 69,6 | 70,7 | 54,9 | 56,4 |
| Sonstige (Mol-%) | 19,2 | 17,8 | 32,5 | 30,2 |
| Temperatur | 253°C | 268°C | 255°C | 264°C |

| Austritt Reaktor (8): | | | | |
|---|---|---|---|---|
| CH₃OH (Mol-%) | - | 8,3 | - | 9,4 |
| CO₂ (Mol-%) | - | 2,9 | - | 3,8 |
| CO (Mol-%) | - | 1,0 | - | 1,0 |
| H₂ (Mol-%) | - | 68,0 | - | 52,7 |
| Sonstige (Mol-%) | - | 19,8 | - | 33,1 |
| Temperatur | - | 180°C | - | 186°C |

Im Beispiel 4 ist die gesamte Menge an Katalysator, die auf die Reaktoren (2) und (8) im Verhältnis 42 : 58 verteilt ist, um 15% höher als im Beispiel 3. Der mit siedendem Wasser gekühlte Reaktor (2) des Beispiels 3 hat eine Temperatur des abgeführten Kühlmittels von 250°C und im Beispiel 4 liegt diese Temperatur bei 260°C.

## Patentansprüche

1. Verfahren zum Erzeugen von Methanol aus einem Wasserstoff und Kohlenoxide enthaltenden Synthesegas durch Umsetzung an körnigen, kupferhaltigen Katalysatoren bei Drücken im Bereich von 2 bis 12 MPa (20 bis 120 bar) und Temperaturen im Bereich von 130 bis 350°C, wobei man das Synthesegas durch mindestens zwei in Serie geschaltete Synthese-Reaktoren leitet, dadurch gekennzeichnet,
a) daß man frisches und zurückgeführtes Synthesegas, vorgewärmt auf eine Temperatur im Bereich von 220 bis 280°C, in den ersten Synthese-Reaktor leitet, in welchem der Katalysator in Röhren angeordnet ist, die von unter erhöhtem Druck siedendem Wasser als Kühlmittel umgeben sind,
b) daß man im ersten Synthese-Reaktor 40 bis 80% des eingeleiteten Kohlenoxide umsetzt und ein Gase und Methanoldampf enthaltendes erstes Gemisch abzieht,
c) daß man das erste Gemisch durch mindestens einen zweiten Synthese-Reaktor leitet, in welchem der Katalysator mit Synthesegas gekühlt wird, daß man ein Gase und Methanoldampf enthaltendes zweites Gemisch aus dem zweiten Synthese-Reaktor abzieht, das zweite Gemisch kühlt und Methanoldampf kondensiert und
d) daß man vom methanolhaltigen Kondensat zurückzuführendes Synthesegas abtrennt, das abgetrennte Synthesegas mindestens teilweise als Kühlmittel durch den zweiten Synthese-Reaktor leitet und das dabei vorgewärmte Synthesegas in den ersten Reaktor zurückführt, wobei man dem zurückzuführenden Synthesegas aus einer externen Quelle kommendes frisches Synthesegas zumischt.

2. Verfahren nach Anspruch 1, dadurch gekennzeichent, daß das aus dem ersten Synthese-Reaktor abgezogene erste Gemisch 4 bis 10 Vol. % Methanoldampf enthält.

3. Verfahren nach Anspruch 1 oder 2, dadurch gekennzeichnet, daß das zweite Gemisch mit einer Temperatur von 130 bis 240°C aus dem zweiten Synthese-Reaktor abgezogen wird.

4. Verfahren nach Anspruch 1 oder einem der folgenden, dadurch gekennzeichnet, daß das in den ersten Synthese-Reaktor geleitete Synthesegas zu 15 bis 40 Vol.-% aus frischem Synthesegas besteht.

5. Verfahren nach Anspruch 1 oder einem der folgenden, dadurch gekennzeichnet, daß die im ersten und zweiten Reaktor enthaltenen Katalysatormengen ein Gewichtsverhältnis von 3 : 2 bis 1 : 4 aufweisen.

6. Verfahren nach einem der Ansprüche 1 bis 5, dadurch gekennzeichnet, daß im zweiten Synthese-Reaktor 40 bis 80% der in den zweiten Reaktor eintretenden Kohlenoxide umgesetzt werden.

7. Verfahren nach Anspruch 1 oder einem der folgenden, dadurch gekennzeichnet, daß man pro Stunde und pro m³ Katalysator 14000 bis 24000 Nm³ Synthesegas in den ersten Synthese-Reaktor leitet.

## Claims

1. A process for producing methanol from a synthesis gas containing hydrogen and carbon oxides by reacting on granular, copper-containing catalysts at pressures in the range from 2 to 12 MPa (20 to 120 bar) and temperatures in the range from 130 to 350°C, wherein the synthesis gas is passed through at least two synthesis reactors connected in series, characterised in that
a) fresh and recycled synthesis gas, preheated to a temperature in the range from 220 to 280°C, is passed into the first synthesis reactor, in which the catalyst is arranged in tubes which are surrounded by water which boils at elevated pressure as a coolant,
b) 40 to 80% of the carbon oxides introduced are reacted in the first synthesis reactor and a first mixture containing gases and methanol vapour is withdrawn,
c) the first mixture is passed through at least one second synthesis reactor, in which the catalyst is cooled with synthesis gas, that a second mixture containing gases and methanol vapour is withdrawn from the second synthesis reactor, the second mixture is cooled and methanol vapour is condensed, and
d) synthesis gas which is to be recycled is separated off from the methanol-containing condensate, the synthesis gas separated off is passed at least in part as coolant through the second synthesis reactor and the synthesis gas preheated thereby is recycled into the first reactor, with fresh synthesis gas from an external source being admixed to the synthesis gas which is to be recycled.

2. A process according to Claim 1, characterised in that the first mixture withdrawn from the first synthesis reactor contains 4 to 10% by volume methanol vapour.

3. A process according to Claim 1 or 2, characterised in that the second mixture is withdrawn from the second synthesis reactor at a temperature of 130 to 240°C.

4. A process according to Claim 1 or one of the following claims, characterised in that the synthesis gas passed into the first synthesis reactor consists of 15 to 40% by volume fresh synthesis gas.

5. A process according to Claim 1 or one of the following claims, characterised in that the quantities of catalyst contained in the first and second reactor have a weight ratio of 3 : 2 to 1 : 4.

6. A process according to one of Claims 1 to 5, characterised in that 40 to 80% of the carbon oxides entering the second reactor are reacted in the second synthesis reactor.

7. A process according to Claim 1 or one of the following claims, characterised in that 14,000 to 24,000 sm³ synthesis gas per hour and per m³ catalyst is passed into the first synthesis reactor.

## Revendications

1. Procédé de production de méthanol à partir d'un gaz de synthèse contenant de l'hydrogène et des oxydes de carbone, par réaction sur des catalyseurs en grains renfermant du cuivre, sous des pressions de l'ordre de 2 à 12 MPa (20 à 120 bar) et à des températures de l'ordre de 130 à 350°C, en envoyant le gaz de synthèse dans au moins deux réacteurs de synthèse montés en série, caractérisé en ce que
a) on préchauffe du gaz de synthèse frais et recyclé à une température de l'ordre de 220 à 280°C, on l'envoie dans le premier réacteur de synthèse dans lequel le catalyseur est disposé dans des tubes qui sont entourés en tant qu'agents de refroidissement, d'eau bouillant sous une pression élevée,
b) on fait réagir dans le premier réacteur de synthèse, de 40 à 80 % des oxydes de carbone introduits et on soutire un premier mélange renfermant de la vapeur de méthanol et des gaz,
c) on envoie le premier mélange dans au moins un deuxième réacteur de synthèse dans lequel le catalyseur est refroidi par du gaz de synthèse, on soutire du deuxième réacteur de synthèse un deuxième mélange contenant de la vapeur de méthanol et des gaz, on refroidit le deuxième mélange et on condense la vapeur de méthanol, et
d) on sépare du gaz de synthèse à recycler du condensat contenant du méthanol, on envoie le gaz de synthèse séparé au moins en partie en tant qu'agent de refroidissement dans le deuxième réacteur de synthèse et on retourne au premier réacteur le gaz de synthèse ainsi préchauffé, et on ajoute au gaz de synthèse recyclé du gaz de synthèse frais provenant d'une source extérieure.

2. Procédé suivant la revendication 1, caractérisé en ce que le premier mélange soutiré du premier réacteur de synthèse contient de 4 à 10 % en volume de vapeur de méthanol.

3. Procédé suivant la revendication 1 ou 2, caractérisé en ce que le deuxième mélange est soutiré du deuxième réacteur de synthèse à une température de 130 à 240°C.

4. Procédé suivant la revendication 1 ou l'une des suivantes. caractérisé en ce que le gaz de synthèse envoyé dans le premier réacteur de synthèse est constitué pour 15 à 40 % en volume de gaz de synthèse frais.

5. Procédé suivant la revendication 1 ou l'une des suivantes, caractérisé en ce que les quantités de catalyseurs contenues dans le premier et dans le deuxième réacteur, présentent un rapport pondéral de 3:2 à 1:4.

6. Procédé suivant l'une des revendications 1 à 5, caractérisé en ce que l'on fait réagir dans le deuxième réacteur de synthèse de 40 à 80 % des oxydes de carbone entrant dans le deuxième réacteur.

7. Procédé suivant la revendication 1 ou l'une des suivantes, caractérisé en ce que l'on envoie par heure et par m³ de catalyseur, de 14000 à 24000 m³ dans les conditions normales de température et de pression de gaz de synthèse dans le premier réacteur de synthèse.
